Europäisches Patentamt

European Patent Office

Office européen des brevets

(11) Veröffentlichungsnummer : **0 033 084**
**B1**

# (12) EUROPÄISCHE PATENTSCHRIFT

(45) Veröffentlichungstag der Patentschrift :
16.02.83

(21) Anmeldenummer : **81100190.8**

(22) Anmeldetag : **13.01.81**

(51) Int. Cl.³ : **C 07 C 27/12, C 07 C 29/48,**
**C 07 B 3/00, C 07 C 35/08**

(54) **Verfahren und Vorrichtung zum sicheren Zuführen von molekularem Sauerstoff oder solchen enthaltenden Gasen bei der Oxidation von Kohlenwasserstoffen.**

(30) Priorität : **19.01.80 DE 3001880**

(43) Veröffentlichungstag der Anmeldung :
**05.08.81 Patentblatt 81/31**

(45) Bekanntmachung des Hinweises auf die Patenterteilung : **16.02.83 Patentblatt 83/07**

(84) Benannte Vertragsstaaten :
**BE CH DE FR GB IT LI NL**

(56) Entgegenhaltungen :
**CH A 510 599**
**DE A 1 914 448**
**DE A 2 452 803**
**GB A 977 064**

(73) Patentinhaber : **BASF Aktiengesellschaft**
**Carl-Bosch-Strasse 38**
**D-6700 Ludwigshafen (DE)**

(72) Erfinder : **Rieber, Norbert, Dr.**
**Liebfrauenstrasse 1C**
**D-6800 Mannheim 51 (DE)**
Erfinder : **Helbach, Hans**
**Stettiner Strasse 14**
**D-6840 Lampertheim (DE)**
Erfinder : **Hunziker, Gerhard**
**Glockenloch 10**
**D-6700 Ludwigshafen (DE)**
Erfinder : **Mueller, Guenter**
**Walter-Gropius-Strasse 3**
**D-6700 Ludwigshafen (DE)**

# Verfahren und Vorrichtung zum sicheren Zuführen von molekularem Sauerstoff oder solchen enthaltenden Gasen bei der Oxidation von Kohlenwasserstoffen

Die Oxidation von Kohlenwasserstoffen, z. B. Cyclohexan mit molekularem Sauerstoff in der Flüssigphase wird bei der Herstellung von Cyclohexanol großtechnisch durchgeführt (vgl. CH-PS 510 599). Hierbei erfolgt die Zuführung des molekularen Sauerstoffs in die Reaktionszone, z. B. über Zweistoffdüsen, Einsteckrohre, Fritten oder ähnliche Einrichtungen. Bei der Durchführung solcher Verfahren muß berücksichtigt werden, daß Sauerstoff mit Kohlenwasserstoffen explosive Gemische bildet. Als besonders gefährlich gilt das Eindringen des zu oxidierenden Kohlenwasserstoffs in die Zufuhrleitung des Sauerstoffs oder Sauerstoff enthaltende Gase. Ebenso ist die Ausbildung einer Tauchflamme gefährlich, da dies zum Schmelzen der Zufuhreinrichtungen führt.

Um das Eindringen von Kohlenwasserstoffen in die sauerstoffführenden Leitungen zu verhindern, werden z. B. Druckdifferenz- oder Durchflußgeräte eingesetzt, die beim Unterschreiten eines Minimaldifferenzdruckes oder einer Minimaldurchflußmenge durch Spülung der Leitungen mit Stickstoff die Zufuhr des Sauerstoffs und somit die Reaktion unterbrechen. Diese Arbeitsweisen sind jedoch störanfällig und haben den Nachteil, daß die Meßstellen zur Druck- und Durchflußmessung nur außerhalb des Reaktionsraumes angebracht werden können, wodurch bei plötzlich auftretenden Undichtigkeiten in den Leitungen zwischen den Meßstellen und dem Reaktionsraum die Druckdifferenz oder Durchflußmengenwerte nicht abfallen und so die Zufuhr des Sauerstoffs nicht unterbrochen wird.

Entsprechend der DE-OS 2 452 803 soll an der Austrittsstelle des molekularen Sauerstoffs in das Reaktionsmedium ein Thermoelement angeordnet sein, um die Temperatur zu überwachen un beim Entstehen einer Tauchflamme die Sauerstoffzufuhr zu unterbrechen. Diese Arbeitsweise hat sich zum rechtzeitigen Erkennen des Eindringens von Kohlenwasserstoffen in die sauerstoffzuführende Leitung, bevor es zu einer Explosion oder zur Bildung einer Tauchflamme kommt, nicht bewährt. Bei der Zufuhr von Sauerstoff durch das Einsteckrohr erhitzt sich der molekulare Sauerstoff an den heißen Wänden bis fast auf Reaktionstemperatur. Die durch das Thermoelement angezeigte Temperatur ist deshalb nahezu identisch mit der Reaktortemperatur, so daß sich ein Eindringen von Kohlenwasserstoff in die sauerstoffführende Leitung nicht sofort deutlich durch einen Temperaturanstieg bemerkbar macht. Dies gilt ganz besonders bei der Dosierung von molekularem Sauerstoff über den äußeren Ringspalt von Zweistoffdüsen, wenn man gleichzeitig über die Innenbohrung vorgeheizte Kohlenwasserstoffe zuführt. In diesem Fall erwärmt sich der molekulare Sauerstoff sowohl vom äußeren Teil wie auch vom inneren Teil der Zweistoffdüse.

Es war deshalb die technische Aufgabe gestellt, ein Verfahren und eine Vorrichtung zu entwickeln, die es gestattet, die Zuführung von molekularem Sauerstoff oder solchen enthaltenden Gasen bei der Oxidation von Kohlenwasserstoffen in der flüssigen Phase so zu gestalten, daß bei Eindringen von Kohlenwasserstoffen in die sauerstoffzuführenden Leitungen oder bei der Ausbildung einer Tauchflamme die Oxidation rechtzeitig unterbrochen wird.

Diese Aufgabe wird gelöst in einem Verfahren zur sicheren Zuführung von molekularem Sauerstoff oder solchen enthaltenden Gasen bei der Oxidation von Kohlenwasserstoffen in flüssiger Phase, dadurch gekennzeichnet, daß der zuführende molekulare Sauerstoff, oder das solche enthaltende Gas unmittelbar vor der Stelle des Eintritts in den flüssigen oxidierenden Kohlenwasserstoff eine um mindestens 20 °C niedrigere Temperatur aufweist als die Temperatur des Kohlenwasserstoffs und sobald der zuzuführende molekulare Sauerstoff oder das solchen enthaltende Gas die vorgegebene Temperatur an der Zufuhrstelle überschreitet, die Zufuhr des Sauerstoffs oder des molekularen Sauerstoff enthaltenden Gases unterbrochen wird.

Ferner ist ein Gegenstand der Erfindung eine Vorrichtung zum Einleiten von molekularen Sauerstoff oder solchen enthaltenden Gasen in flüssige kohlenwasserstoffe, gekennzeichnet durch eine rohrförmige Ummantelung 1 mit einer Austrittsdüse 2, ein in Längsrichtung in der Ummantelung durch Abstandshalter 3 im Abstand von der Ummantelung angeordnete Zuführungsrohr 4 für molekularen Sauerstoff oder solchen enthaltende Gase mit der Maßgabe, daß das Zuführungsrohr 2 innerhalb der Ummantelung 1 vor der Austrittsdüse 4 endet und zwischen dem Ende des Zuführungsrohres 4 und der Austrittsdüse 2 ein Element 5 zur Temperaturmessung angeordnet ist.

Das neue Verfahren und die neue Vorrichtung haben den Vorteil, daß das Eindringen von Kohlenwasserstoffen in die sauerstofführenden Zuführungsleitungen sicher zu erkennen ist und die Ausbildung einer Tauchflamme vermieden wird.

Das erfindungsgemäße Verfahren eignet sich beispielsweise für die Oxidation von Cycloalkanen zu Cycloalkylhydroperoxiden oder zu Cycloalkanolen und Cycloalkanonen in flüssiger Phase ferner für die Direktoxidation von Propylen zu Propylenoxid, sowie für die Co-oxidation von Kohlenwasserstoffen, z. B. für die Herstellung von Propylenoxid durch gemeinsame Oxidation von Propylen und Äthylbenzol. Besondere technische Bedeutung hat das erfindungsgemäße Verfahren bei der Oxidation von Cyclohexan wie es beispielsweise in der CH-PS 5 105 599 beschrieben ist, erlangt. In der Regel wird die Oxidation von Cyclohexan bei Temperaturen von 140 bis 200 °C unter Drücken von 10 bis 60 bar gegebenenfalls unter Mitverwendung von Katalysatoren, wie Kobaltsalze, durch Einleiten von molekularem Sauerstoff oder solchen enthaltenden Gasen in das flüssige Reaktionsgemisch durchgeführt.

Erfindungsgemäß verfährt man so, daß der zugeführte molekulare Sauerstoff oder das molekularen Sauerstoff enthaltende Gas unmittelbar vor der Stelle des Eintritts in den flüssigen heißen Kohlenwasserstoff, d. h. an der Düsenöffnung eine um mindestens um 20° niedrigere Temperatur aufweist als die Temperatur des Kohlenwasserstoffs. Wird beispielsweise Cyclohexan bei einer Temperatur von 180 °C oxidiert, so soll der zugeführte molekulare Sauerstoff kurz vor der Düsenöffnung eine Temperatur von 160 °C nicht überschreiten. Dies erreicht man vorteilhaft, indem man bei Zuführungsleitungen für den molekularen Sauerstoff, die in das heiße Reaktionsgemisch hineinragen, die Wärmeübertragung vom heißen Reaktionsgemisch auf den zuzuführenden molekularen Sauerstoff vermeidet oder vermindert. Besonders bewährt hat es sich, die in das heiße Reaktionsgemisch hineinragende Zuführungsleitung für molekularen Sauerstoff durch eine isolierende Gasschicht zu umgeben. Dies erreicht man auf einfache Weise, indem man die in das heiße Reaktionsgemisch hineinragende Zuführungsleitung für molekularen Sauerstoff mit einem weiteren Rohr umgibt, dessen Austrittsende etwas eingeengt sein kann mit der Maßgabe, daß Zuführungsleitung für den molekularen Sauerstoff vorher endet. Hierdurch ergibt sich bei Zuführen von Sauerstoff eine Gasschicht, die isolierend wirkt. Im Zwischenraum zwischen dem Austritt des molekularen Sauerstoffs aus der Zuführungsleitung und der Öffnung der Ummantelung wird die Temperatur des molekularen Sauerstoffs gemessen. Sobald der zuzuführende molekulare Sauerstoff die vorgegebene Temperaturen der Austrittsöffnung überschreitet, wird die Zufuhr des molekularen Sauerstoffs oder des molekularen Sauerstoff enthaltenden Gases unterbrochen. Zweckmäßig wird die Zuführungsleitung dann sofort automatisch mit Stickstoff oder einem anderen in Inertgas gespült. Auf diese Weise ist sichergestellt, daß keine unkontrollierte Reaktion in der Zuführungsleitung für den molekularen Sauerstoff oder die Ausbildung einer Tauchflamme eintritt. Vorteilhaft läßt sich die Temperatur des molekularen Sauerstoffs an der Austrittsstelle auch noch durch die Wahl des Querschnitts für die Zuführungsleitung und Strömungsgeschwindigkeit des molekularen Sauerstoffs beeinflussen. Diese Parameter sind durch einfache Vorversuche leicht zu ermitteln.

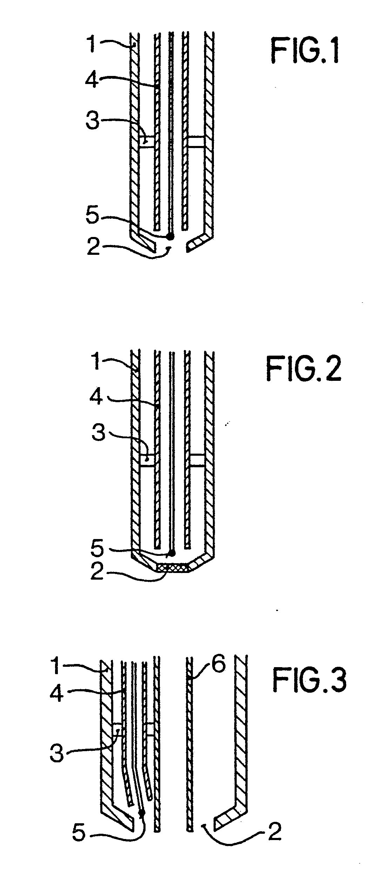

Eine geeignete Vorrichtung zur Durchführung des Verfahrens wird beispielsweise in Fig. 1 beschrieben. Die Vorrichtung besteht aus einer rohrförmigen Ummantelung 1 mit einer zweckmäßig verengten Austrittsdüse 2 für die einzuleitenden Medien. In Längsrichtung ist in der Ummantelung durch Abstandshalter 3 ein Zuführungsrohr 4 für molekularen Sauerstoff oder solche enthaltende Gase im Abstand von der Ummantelung angeordnet. Das Zuführungsrohr 4 endet innerhalb der Ummantelung 1 vor der Austrittsdüse 2. Hierdurch wird beim Einleiten von Sauerstoff durch das Zuführungsrohr 4 der Zwischenraum zwischen dem Zuführungsrohr 4 und der Ummantelung 1 von selbst mit Sauerstoff gefüllt und es bildet sich eine isolierende Gasschicht aus. Zwischen dem Ende des Zuführungsrohres 4 und der Austrittsdüse 2 ist ein Element 5 zur Temperaturmessung angeordnet. Ein geeignetes Element zur Temperaturmessung ist beispielsweise ein NI/CrNi-Thermoelement. Zweckmäßig führt man das Element 5 zur Temperaturmessung über das Zuführungsrohr 4 zu, um eine möglichst geringe Beeinflussung durch das heiße Reaktionsmedium zu gewährleisten.

Eine weitere Ausgestaltung wird in Fig. 2 gezeigt, in der 1 eine rohrförmige Ummantelung, 2 eine mit einer Fritte versehene Austrittsdüse, 3 Abstandshalter, 4 ein Zuführungsrohr und 5 ein Element zur Temperaturmessung bezeichnen. Anstatt einer Fritte können auch eine Vielzahl von Düsen verwendet werden.

Eine weitere Ausführungsform wird in der Zweistoffdüse gemäß Fig. 3 gezeigt, in der 1 eine rohrförmige Ummantelung, 2 eine Austrittsöffnung für die einzuleitenden Medien, 3 Abstandshalter und (4) ein Zuführungsrohr für molekularen Sauerstoff oder solchen enthaltende Gase, 5 ein Element zur Temperaturmessung und 6 ein Zuführungsrohr für Kohlenwasserstoffe bedeuten.

## Ansprüche

1. Verfahren zur sicheren Zuführung von molekularem Sauerstoff oder solchen enthaltenden Gasen bei der Oxidation von Kohlenwasserstoffen in flüssiger Phase, dadurch gekennzeichnet, daß der zuzuführende molekular Sauerstoff oder das solche enthaltende Gas unmittelbar vor der Stelle des Eintritts in flüssigem Kohlenwasserstoff eine um mindestens 20° niedrigere Temperatur aufweist, als die Temperatur des Kohlenwasserstoffs und sobald der zuzuführende molekulare Sauerstoff oder das solche enthaltende Gas die vorgegebene Temperatur an Zufuhröffnung überschreitet, die Zufuhr des molekularen Sauerstoffs oder des molekularen Sauerstoffs enthaltenden Gases unterbrochen wird.

2. Verfahren nach Anspruch 1, dadurch gekennzeichnet, daß der zuzuführende molekulare Sauerstoff oder das molekularen Sauerstoff enthaltende Gas eine um 20 bis 150° niedrigere Temperatur aufweist als die Temperatur des Kohlenwasserstoffs.

3. Vorrichtung zum Einleiten von molekularem Sauerstoff oder solchen enthaltendem Gas in flüssige Kohlenwasserstoffe, gekennzeichnet durch eine rohrförmige Ummantelung 1 mit einer Austrittsdüse 2, ein in Längsrichtung in der Ummantelung durch Abstandshalter 3 im Abstand von der Ummantelung angeordnetes Zuführungsrohr 4 für molekulareren Sauerstoff oder solchen enthaltende Gase, das innerhalb der Ummantelung 1 vor der Austrittsdüse 2 endet und ein zwischen dem Ende des Zuführungsrohres 4 und der Austrittsdüse 2 angeordnetes Element 5 zur Temperaturmessung.

4. Vorrichtung nach Anspruch 3, dadurch gekennzeichnet, daß die Austrittsöffnung 2 als Fritte ausgebildet ist, oder aus mehreren Düsen besteht.

5. Vorrichtung nach Anspruch 3, dadurch gekennzeichnet, daß in der Ummantelung 1 in Längsrichtung zusätzlich ein Zuführungsrohr 6 für Kohlenwasserstoffe angeordnet ist.

## Claims

1. A process for the safe supply of molecular oxygen or gas containing molecular oxygen in the oxidation of hydrocarbons in the liquid phase, wherein the molecular oxygen, or gas containing molecular oxygen, which is to be supplied is, immediately before the point of entry into the liquid hydrocarbon, at least 20 °C colder than the hydrocarbon, and wherein, as soon as the molecular oxygen, or gas containing molecular oxygen which is to be supplied exceeds the predetermined temperature at the supply orifice, the supply of molecular oxygen or gas containing molecular oxygen is interrupted.

2. A process as claimed in claim 1, wherein the molecular oxygen, or gas containing molecular oxygen, which is to be supplied is from 20° to 150 °C colder than the hydrocarbon.

3. Apparatus for introducing molecular oxygen, or a gas containing molecular oxygen, into a liquid hydrocarbon, which comprises a tubular jacket 1 with an exit nozzle 2 and a supply tube 4 for molecular oxygen, or gas containing molecular oxygen, which is located lengthwise in the jacket and is kept at a distance therefrom by spacers 3, said tube terminating within the jacket 1 before the exit nozzle 2, and an element 5, for temperature measurement, located between the end of the supply tube 4 and the exit nozzle 5.

4. Apparatus as claimed in claim 3, wherein the exit orifice 2 is in the form of a frit or consists of a plurality of nozzles.

5. Apparatus as claimed in claim 3, wherein a supply tube 6 for hydrocarbons is additionally located lengthwise within the jacket 1.

## Revendications

1. Procédé pour l'introduction sûre d'oxygène moléculaire ou d'un mélange gazeux en contenant lors de l'oxydation en phase liquide d'hydrocarbures, caractérisé en ce que, à l'endroit d'introduction dans l'hydrocarbure liquide, l'oxygène moléculaire ou le mélange gazeux en contenant se trouve à une température inférieure d'au moins 20 °C à la température de l'hydrocarbure et, dès que l'oxygène moléculaire ou le mélange gazeux en contenant dépasse le seuil de la température maximale fixée au point d'introduction, l'amenée de l'oxygène moléculaire ou du mélange gazeux en contenant est interrompue.

2. Procédé suivant la revendication 1, caractérisé en ce que l'oxygène moléculaire ou le mélange gazeux en contenant à introduire se trouve à une température inférieure de 20 à 150 °C à la température de l'hydrocarbure.

3. Dispositif pour l'introduction d'oxygène moléculaire ou d'un mélange gazeux en contenant dans des hydrocarbures liquides, caractérisé en ce qu'il se compose d'une enveloppe tubulaire extérieure 1, se terminant par une extrémité 2 formant tuyère, d'un tuyau d'amenée 4 de l'oxygène moléculaire ou du mélange gazeux en contenant, maintenu à une certaine distance de l'enveloppe tubulaire extérieure 1 par des éléments d'espacement 3 et se terminant, à l'intérieur de l'enveloppe tubulaire 1, avant l'extrémité formant tuyère 2, ainsi que d'un élément de contrôle de la température 5, situé entre l'extrémité 4 du tuyau d'introduction 4 et l'extrémité 2 formant tuyère de l'enveloppe tubulaire 1.

4. Dispositif suivant la revendication 3, caractérisé en ce que l'extrémité 2 formant tuyère est réalisée sous forme d'une fritte ou de celle de plusieurs tuyères ou buses.

5. Dispositif suivant la revendication 3, caractérisé en ce que, à l'intérieur de l'enveloppe tubulaire extérieure 1, est en outre disposé un tube concentrique 6 pour l'amenée de l'hydrocarbure.

FIG.1

FIG.2

FIG.3